# EUROPEAN PATENT APPLICATION

(11) **EP 4 635 475 A1**
(43) Date of publication of application: **22.10.2025**
(21) Application number: 24170369.3
(22) Date of filing: 15.04.2024
(51) Int. Cl.: A61K 8/99, A61Q 19/00, A61Q 19/08

(54) **COSMETICAL COMPOSITION COMPRISING BACTERIAL COMPONENTS**

(71) Applicant: CLR-Chemisches Laboratorium Dr. Kurt Richter GmbH, 12277 Berlin (DE)
(72) Inventor: The designation of the inventor has not yet been filed
(74) Representative: Eisenführ Speiser

(57) **Abstract**

The present invention generally relates to the field of cosmetics. More particularly, the invention relates to a cosmetic skin care composition comprising components of bacterial cells belonging to the species of *Lactobacillus kefiranofaciens.* The invention is based on the finding that such components have favorable effects on skin. The invention therefore also provides a cosmetic method of improving skin appearance, improving skin elasticity, reducing transepidermal water loss or preventing the signs of aging in a subject, comprising administering a skin care composition of the present invention.

## Description

The present invention generally relates to the field of cosmetics. More particularly, the invention relates to a cosmetic skin care composition comprising components of bacterial cells belonging to the species of *Lactobacillus kefiranofaciens.* The invention is based on the finding that such components have favorable effects on skin. The invention also provides a cosmetic method of improving skin appearance, improving skin elasticity, reducing transepidermal water loss or preventing the signs of aging in a subject, comprising administering a skin care composition of the present invention.

### BACKGROUND OF THE INVENTION

Cosmetic products for topical application that improve the appearance of skin are strongly in demand. These products shall soften and moisturize the skin in order to delay the signs of stress and aging. In recent years, a number of chemical compounds that were commonly used in cosmetic products have been replaced by natural ingredients, thereby following the consumers' demand for more natural products.

For example, plant and herbal extracts are now commonly used as additives in skin care products. Some plant extracts have been found to exert highly beneficial effects to skin. For example, extracts of Epilobium angustifolium have been found to sooth the skin. Other extracts, such as extracts from *Avena sativa,* are known to relieve the skin from itching and irritation. Extracts of the bark of *Prunus serotina* were found to strongly moisturize the skin. Accordingly, plant or herbal extracts are widely used by the cosmetic industry due to their wide variety of skin improving properties.

Another group of compounds that has gained considerable attention in the last years are bacterial ferments and lysates. For example, ferments and lysates from different bacterial species, such as *Lactococcus, Lactobacillus, Streptococcus* and *Bacillus,* have been used as additives in cosmetics based on their anti-aging, skin whitening, anti-inflammatory, and photoprotective effects.

Despite the progress that has been made in the field of cosmetics in the past in identifying and developing effective ingredients for skin care products, there is still a need for additional compounds that can be used for improving skin appearance.

### DISCLOSURE OF THE INVENTION

The present invention is based on the finding that non-viable components of bacterial cells belonging to the bacterial species of *Lactobacillus kefiranofaciens,* and in particular lysates of *L. kefiranofaciens,* are highly suitable for being used in skin products. Specifically, it has been found by the inventors that non-viable bacterial components which are derived from this particular species effectively reduce the transepidermal water loss in the skin and at the same time increase the antioxidative potential of the skin. In this way, these non-viable bacterial components are highly effective in improving skin appearance and skin elasticity and preventing the signs of aging.

In a first aspect, the present invention provides a cosmetic skin care composition comprising, as an active ingredient, non-viable components of bacterial cells belonging to the species of *L*. kefiranofaciens. This means that the skin care composition of the invention does not include any living bacterial cells, but instead only components that have been derived from killed or inactivated bacteria. Such components which include metabolic products like polysaccharides, proteins and lipids, are sometimes referred to in the literature as "post-biotics".

The species *L*. kefiranofaciens includes homofermentative, rod-shaped lactic acid bacteria that were originally isolated from kefir grains. In a preferred embodiment, the bacterial cell components are derived *L. kefiranofaciens subsp. kefiranofaciens.* Suitable strains include, for example, strain ZW3 identified by Wang et al. [4]. Another suitable strain is LMG 19149^{T} which is described in references [5]-[7]. Yet another suitable strain is WT-8 which is described in reference [5] and which is deposited at the Leibniz Institute DSMZ-German Collection of Microorganisms and Cell Cultures GmbH (Braunschweig, Germany) under deposit number DSM 5017.

The identification of *L. kefiranofaciens* and its subspecies can be achieved by different methods known in the art. Suitable methods for the discrimination of different Lactobacillus species include, for example, either phenotype-based methods, such as whole cell protein electrophoretic profiles by SDS-PAGE and whole bacteria compounds by infrared spectroscopy, and molecular methods techniques, such as random amplified polymorphic DNA (RAPD), sequence-based identification using phenylalanyl-tRNA synthase gene (pheS), repetitive element palindromic PCR fingerprinting (rep-PCR) using the (GTG)5 primer, 16S rRNA gene sequencing, and colony PCR. These methods are described in reference [9]. In a preferred aspect, the identification of *L. kefiranofaciens* and its subspecies is performed by 16S rRNA gene sequencing. This method has been summarized in reference [10].

In a particularly preferred embodiment of the invention, the non-viable bacterial components which are included in the cosmetic skin care composition of the invention have been derived from a *L. kefiranofaciens* strain that produces kefiran, an exopolysaccharide that comprises a high number of hydroxyl groups. This branched glucogalactan, which consists of roughly equal amounts of D-glucose and D-galactose, has recently attracted attention due to its beneficial rheological behavior. Thus, in a preferred embodiment, the cosmetic skin care composition of the invention comprises a certain level of the bacterial exopolysaccharide kefiran. Kefiran can be detected and quantified by using the Antrona method which is described in reference [8]. Alternatively, the presence of kefiran in the cosmetic skin care composition of the invention can be detected by using an anti-kefiran antibody.

In another preferred embodiment, the non-viable bacterial components are included in the cosmetic skin care composition of the invention in form of a lysate or, more preferably, a ferment lysate. As used herein, a "lysate" refers to a composition obtained by destruction or dissolution of bacterial cells via cell lysis. By lysing the bacterial cell, all intracellular constituents formerly contained in the bacterial cells are released. Lysis can be effected by various well-known methods, including high pressure homogenization, ultrasonication, heat-treatment, osmotic shock, mechanical stress, enzymatic degradation or irradiation. It is preferred that the lysate used in the cosmetic skin care composition of the invention has been obtained by high pressure homogenization.

Preferably, the lysate used in the cosmetic skin care composition of the invention is a "ferment lysate" which means that the lysate is obtained from a fermentation culture, i.e. a culture in which the respective *L. kefiranofaciens* strain has been used for fermenting a substrate.

In another particularly preferred embodiment, the microbial cell debris is removed prior to use of the lysate by filtration. A *L. kefiranofaciens* lysate which has been subjected to filtration is referred to herein as lysate filtrate. Similarly, a *L. kefiranofaciens* ferment lysate which has been subjected to filtration is referred to herein as ferment lysate filtrate. In one embodiment, the lysate used in the cosmetic skin care composition of the invention is a lysate filtrate, and more preferably a ferment lysate filtrate.

Therefore, in a preferred aspect, the invention relates to a cosmetic skin care composition comprising a lysate that has been derived from a *L. kefiranofaciens* culture, and more preferably a *L. kefiranofaciens subsp. kefiranofaciens* culture. In another preferred aspect, the invention relates to a cosmetic skin care composition comprising a lysate filtrate that has been derived from a *L. kefiranofaciens* culture, and more preferably a *L. kefiranofaciens subsp. kefiranofaciens* culture.

In yet another preferred aspect, the invention relates to a cosmetic skin care composition comprising a ferment lysate that has been derived from a *L. kefiranofaciens* culture, and more preferably a *L. kefiranofaciens subsp. kefiranofaciens* culture. In yet another preferred aspect, the invention relates to a cosmetic skin care composition comprising a ferment lysate filtrate that has been derived from a *L. kefiranofaciens* culture, and more preferably a *L. kefiranofaciens subsp. kefiranofaciens* culture.

In yet another preferred aspect, the invention relates to a cosmetic skin care composition comprising a lysate that has been derived from a kefiran-producing *L. kefiranofaciens* culture, and more preferably a *L. kefiranofaciens subsp. Kefiranofaciens* culture. In another preferred aspect, the invention relates to a cosmetic skin care composition comprising a lysate filtrate that has been derived from a kefiran-producing *L. kefiranofaciens* culture, and more preferably a *L. kefiranofaciens subsp. kefiranofaciens* culture.

In yet another preferred aspect, the invention relates to a cosmetic skin care composition comprising a ferment lysate that has been derived from a kefiran-producing *L. kefiranofaciens* culture, and more preferably a *L. kefiranofaciens subsp. kefiranofaciens* culture. In yet another preferred aspect, the invention relates to a cosmetic skin care composition comprising a ferment lysate filtrate that has been derived from a kefiran-producing *L. kefiranofaciens* culture, and more preferably a *kefiranofaciens subsp. kefiranofaciens* culture.

A ferment lysate of *L. kefiranofaciens* strains can be prepared in accordance with methods that have been described extensively in the prior art. In a first step, the *L. kefiranofaciens* strain that is selected for lysate preparation is cultured in a suitable medium. Suitable culture media that allow for the propagation of *L. kefiranofaciens* are described in the prior art and also in the below example part of the application. It is preferred that the culturing is performed under anaerobic conditions, e.g., in a N₂ atmosphere. The temperature during culturing can be between about 32-38°C and will preferably be about 32°C. Batch culturing is continued until the glucose in the culture medium is depleted. Glucose depletion can be measured by a glucose sensor during cell culturing. Depending on the amount of glucose used in the medium and the inoculation amount, cell culturing can last for several days until the glucose is depleted. Culturing can include gentle stirring, e.g., with a magnetic stirrer.

Once the glucose is depleted, the cell number of in the culture is preferably adapted to 1×10⁶ cells per ml (cells/ml) to 1×10¹¹ cells/ml, and more preferably 1×10⁷ cells/ml to 1×10¹⁰ cells/ml, and most preferably 1×10⁸ cells/ml to 1×10⁹ cells/ml, either by diluting the or concentrating the culture. Concentration of the culture can be achieved by centrifugation or filtration. The cell number can be determined by routine methods and is preferably determined by use of a Neubauer chamber.

The cells may be washed before lysis, but preferably no washing step is performed.

A heat inactivation step may be performed by heating the content of the fermenter to 85-90°C for at least 5 minutes, and preferably for a period of 30-60 minutes. Heat inactivation may also be performed before cell counting.

The cells are then disrupted by a suitable method, such as high pressure homogenization, ultrasonication, heat-treatment, osmotic shock, mechanical stress, enzymatic degradation or irradiation. For example, the cells may be subjected to several cycles of a high pressure homogenization device. The entire content of a fermenter, or parts of it, can be led through the homogenization device in several batches to effect cell disruption. The resulting homogenate can be filtrated, e.g. by ultrafiltration, to concentrate the lysate or defined components in the lysate. The lysates can then be stored until further use.

Generally, a skilled person working in the field of formulating cosmetics will be readily able to add the bacterial cell components, e.g. the lysate, in suitable amounts to the final skin care composition. Preferably, the bacterial cell components are present in the cosmetic skin care composition in an amount of 0.1 to 15.0% (w/w). This means that the bacterial cell components can be present in the cosmetic skin care composition in a range from about 0.1 to about 12.5% (w/w), from about 0.1 to about 10.0% (w/w), from about 0.1 to about 7.5% (w/w), from about 0.1 to about 5.0% (w/w), from about 0.1 to about 4.5% (w/w), from about 0.1 to about 4.0% (w/w), from about 0.1 to about 3.5% (w/w), from about 0.1 to about 3.0% (w/w), from about 0.1 to about 2.5% (w/w), from about 0.1 to about 2.0% (w/w), from about 0.1 to about 1.5% (w/w), from about 0.1 to about 1.0% (w/w), from about 0.1 to about 0.5% (w/w), from about 0.5 to about 15.0% (w/w), from about 0.5 to about 12.5% (w/w), from about 0.5 to about 10.0% (w/w), from about 0.5 to about 7.5% (w/w), from about 0.5 to about 5.0% (w/w), from about 0.5 to about 4.5% (w/w), from about 0.5 to about 4.0% (w/w), from about 0.5 to about 3.5% (w/w), from about 0.5 to about 3.0% (w/w), from about 0.5 to about 2.5% (w/w), from about 0.5 to about 2.0% (w/w), from about 0.5 to about 1.5% (w/w), from about 0.5 to about 1.0% (w/w), from about 1.0 to about 15.0% (w/w), from about 1.0 to about 12.5% (w/w), from about 1.0 to about 10.0% (w/w), from about 1.0 to about 7.5% (w/w), from about 1.0 to about 5.0% (w/w), from about 1.0 to about 4.5% (w/w), from about 1.0 to about 4.0% (w/w), from about 1.0 to about 3.5% (w/w), from about 1.0 to about 3.0% (w/w), from about 1.0 to about 2.5% (w/w), from about 1.0 to about 2.0% (w/w), or from about 1.0 to about 1.5% (w/w). The above percentages refer to a lysate, e.g. a ferment lysate, with has been derived from a cell suspension of 1×10⁸ cells/ml to 1x19 cells/ml of *L. kefiranofaciens* cells.

As used herein, all percentages are weight percentages which are based on the overall weight of the cosmetic skin care composition, unless specifically stated otherwise.

The lysate can be directly used for formulating the skin care composition of the invention. It is however preferred to keep the lysate for several hours at ambient temperature for 1-24 hours, preferably for 8-16 hours, in order to stabilize the lysate.

The cosmetic skin care composition of the invention is preferably formulated for topical application which means that the composition is provided in a form that allows the consumer to dispense the composition after application to the skin. Preferably, the skin care composition of the invention is formulated as a liquid, ointment, cream, scrub, lotion, paste, gel, hydrogel, foam, or powder. Where the composition is formulated as a liquid, it can be packaged into a pump dispenser which allows spraying the liquid onto the skin areas to be treated. The skin care composition of the invention can also be incorporated into a patch which is applied to the skin.

The skin area to be treated may include the skin of the face and the body, e.g. the skin of the neck, chest, back, arms, hands, legs or thighs. According to a preferred embodiment, the skin to be treated with the composition of the present invention is the skin of the face. According to another preferred embodiment, the skin to be treated with the composition of the present invention is the skin of the body.

The skin care compositions may be applied to the area of skin in need of treatment, e.g. the face or body, at least once a day, twice a day, or even more frequently if needed. When applied twice daily, the first and second applications are preferably separated by at least 6 hours, preferably 8 hours. Typically, the cosmetic composition is applied once in the morning and once in the evening. The composition of the invention can be used over long periods without any adverse side effects. For example, the period of treatment may be at least 1 week, at least 2 weeks, at least 3 weeks, at least 4 weeks, at least 6 weeks, at least 12 weeks, at least 24 weeks, or more. In some embodiments, the treatment will be extended for several months, such as for 4 months, 6 months, 8 months, 12 months, 18 months, or 24 months.

Apart from the bacterial cell components, the skin care composition of the invention will comprise at least one dermatologically acceptable carrier. The carrier may be present in the skin care composition in an amount of about 30% to about 99%, preferably from about 40% to about 98%, more preferably from about 50% to about 97%, more preferably from about 60% to about 96%, and even more preferably from about 70% to about 95%, by weight of the skin care composition of the invention. As used herein, a dermatologically acceptable carrier is compatible both with the skin and the other ingredients in the composition. In particular, a dermatologically acceptable carrier will not irritate or otherwise affect the skin adversely. Also, a dermatologically acceptable carrier will not interfere with the activity or solubility or dispersibility of other ingredients in the composition. Suitable dermatologically acceptable carriers include, but are not limited to, aqueous solutions, emulsions, dispersions, and solids. In a preferred embodiment, the dermatologically acceptable carrier is an emulsion. The emulsion may be oil-in-water emulsion, a water-in-oil emulsion, or a water-in-oil-in-water emulsion. When an emulsion is used as a carrier, it is preferred that the aqueous phase of the emulsion comprises the bacterial cell components. The emulsion may contain one or more anionic, cationic or non-ionic emulsifier, e.g., in an amount of 1% to about 5% based on the weight of the carrier.

The composition of the invention may further comprise additional optional ingredients that are commonly used in cosmetic skin care products. Such ingredients are described in detail, for example, in the International Cosmetic Ingredient Dictionary and Handbook, 16^{th} ed. (2016) . For example, the composition of the invention may also include one or more of the following: antioxidants, binders, pH adjusters, buffering agents, colorants, thickeners, emollients, humectants, exfoliating agents, preservatives, plant extracts, essential oils, and fragrances.

For example, the composition of the invention may comprise an exfoliating compound such as urea. Exfoliating compounds which are useful in the compositions of the present invention include, but are not limited to, urea, alpha-hydroxy acids and beta-hydroxy acids, and their esters, anhydrides, and salts. Suitable hydroxy acids include, for example, urea, glycolic acid, lactic acid, malic acid, mandelic acid, tartaric acid, citric acid, 2-hydroxyalkanoic acid, salicylic acid, and derivatives thereof. The use of urea is particularly preferred, because it also has an additional water-binding effect and enhances the absorption of other cosmetic ingredients. The skin care composition of the invention may comprise from about 1% to about 5% (w/w) of the exfoliating compound. In some embodiments, the composition may contain two or more different exfoliating compounds.

In one preferred embodiment, the skin care composition of the present invention comprises a compound that serves as a pH adjuster. Since the composition of the invention is used on the human skin, it will normally have a slightly acidic pH to make it more compatible with the acidic environment of the skin. The composition may have a pH in the range from about 2.5 to about 6.5, preferably from about 4.0 to about 6.0, and more preferably from about 5.0 to about 6.0 or from about 5.5 to about 6.0. The acidic pH can be achieved by adding an acid to the skin care composition of the invention, e.g. a carboxylic acid, such as an alpha hydroxy acid. The nature of the acid that can be used in the composition of the invention is not particularly limited. Suitable acids include formic acid, acetic acid, propionic acid, butyric acid, valeric acid, caproic acid, enanthic acid, caprylic acid, and the like. In a particular preferred embodiment, the composition comprises lactic acid as a pH adjuster. For application to the human skin, lactic acid is particularly useful, as it is also secreted by the skin flora to form the protective acidic milieu on the human skin surface.

The skin care composition of the present invention may also comprise an emollient. Suitable emollients for the skin care composition of the present invention include, but are not limited to, olive oil, palm oil, soybean oil, sesame seed oil, rapeseed oil, evening primrose oil, sunflower seed oil, avocado oil, olive oil, coconut oil, castor oil, safflower seed oil, myristyl lactate, isopropyl myristate, polyethylene glycol, isopropyl palmitate, isopropyl stearate, isobutyl palmitate, isocetyl stearate, or cetyl alcohol. The skin care composition of the invention may comprise from about 1% to about 5% (w/w) of the emollient. In some embodiments, the composition may contain two or more different emollients.

The skin care composition of the present invention may also comprise a humectant for improving skin hydration. Suitable humectants for use in the composition of the present invention include, but are not limited to, glycerine, polyethylene glycol ethers of glycerine, amino acids, such as proline and arginine, sugar and sugar alcohols, such as glucose, mannose, trehalose, and polyglycerol sorbitol, 1,3-butylene glycol, propylene glycol, diglycerol, glycerol monopropoxylate, glycogen, sodium hyaluronate, sodium poly-aspartate, sodium poly-glutamate, sorbeth 20, sorbeth 6, and hydrogenated starch hydrolysates. The skin care composition of the invention may comprise from about 1% to about 5% (w/w) of the humectant. In some embodiments, the composition may contain two or more different humectants.

It is preferred that the skin care composition of the present invention comprises at least about 30% (w/w) water. More preferably, the skin care composition comprises at least about 35% (w/w) water, at least about 40% (w/w) water, at least about 45% (w/w) water, at least about 50% (w/w) water, at least about 55% (w/w) water, at least about 60% (w/w) water, at least about 65% (w/w) water, at least about 70% (w/w) water, at least about 75% (w/w) water, at least about 80% (w/w) water, at least about 85% (w/w) water, or at least about 90% (w/w) water. An amount of at least about 70% (w/w) water in the final skin care composition is most preferred.

In another aspect, the present invention provides a cosmetic method of improving skin appearance in a subject, comprising administering a composition as described elsewhere herein to the skin of said subject, preferably the facial skin.

In yet another aspect, the present invention provides a cosmetic method of improving skin elasticity, comprising administering a composition of any of claims 1-8 to the skin of said subject, preferably the facial skin.

In yet another aspect, the present invention provides a cosmetic method of reducing transepidermal water loss, comprising administering a composition of any of claims 1-8 to the skin of said subject, preferably the facial skin.

In yet another aspect, the present invention provides a cosmetic method of preventing the signs of aging, comprising administering a composition of any of claims 1-8 to the skin of said subject, preferably the facial skin.

In yet another aspect, the present invention provides a cosmetic method of improving skin appearance in a subject, comprising administering a composition as described elsewhere herein to the skin of said subject. In yet another aspect, the present invention provides a cosmetic method of improving skin elasticity, comprising administering a composition as described elsewhere herein to the skin of said subject. In yet another aspect, the present invention provides a cosmetic method of reducing transepidermal water loss, comprising administering a composition as described elsewhere herein to the skin of said subject. In yet another aspect, the present invention provides a cosmetic method of preventing the signs of aging, comprising administering a composition as described herein to the skin of said subject.

In a particular preferred embodiment, the above methods comprise the topical administration of the composition to the facial skin.

In another aspect, the present invention provides for the use of a composition as described elsewhere herein, i.e. a composition comprising components of bacterial cells belonging to the species of *L. kefiranofaciens* as an active ingredient, for cosmetic skin care. Specifically, the composition is used for improving skin appearance in a subject, improving skin elasticity, reducing transepidermal water loss, or preventing the signs of aging, comprising administering a composition as described herein to the skin of said subject, preferably the facial skin.

In another aspect, the present invention provides for the use of a composition as described elsewhere herein, i.e. a composition comprising components of bacterial cells belonging to the species of *Lactobacillus kefiranofaciens* as an active ingredient, for cosmetic skin care.

### BRIEF DESCRIPTION OF THE FIGURES

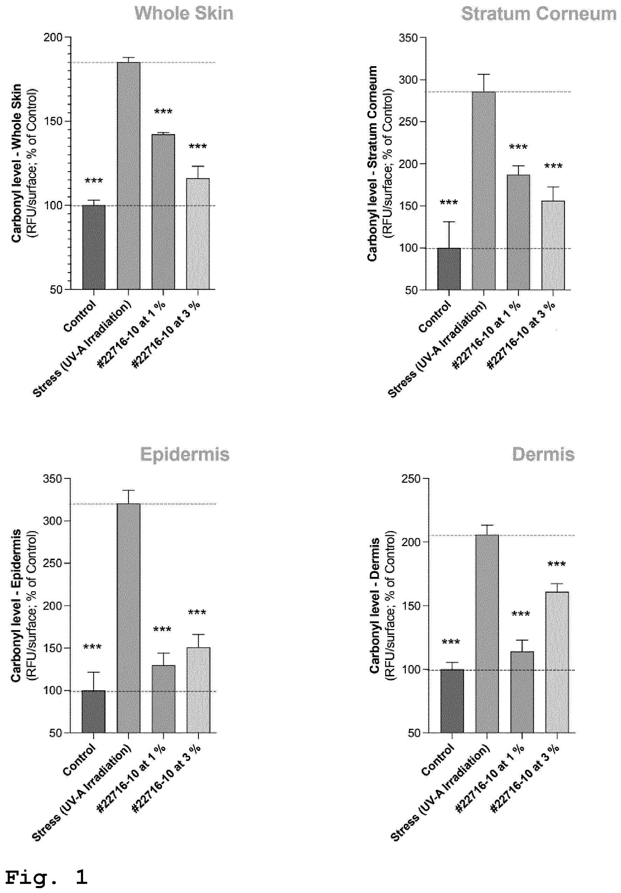

Fig. 1 shows the results of the quantification of carbonylated protein levels. The levels of carbonyls of each experimental group are expressed as relative values (% vs Control) and shown as mean +/- S.D. ***p<0.001; ** p<0.01; - one-way ANOVA and Dunnett's post-hoc test for multi-comparisons vs Stress (UV-A) group (alpha=0.05).

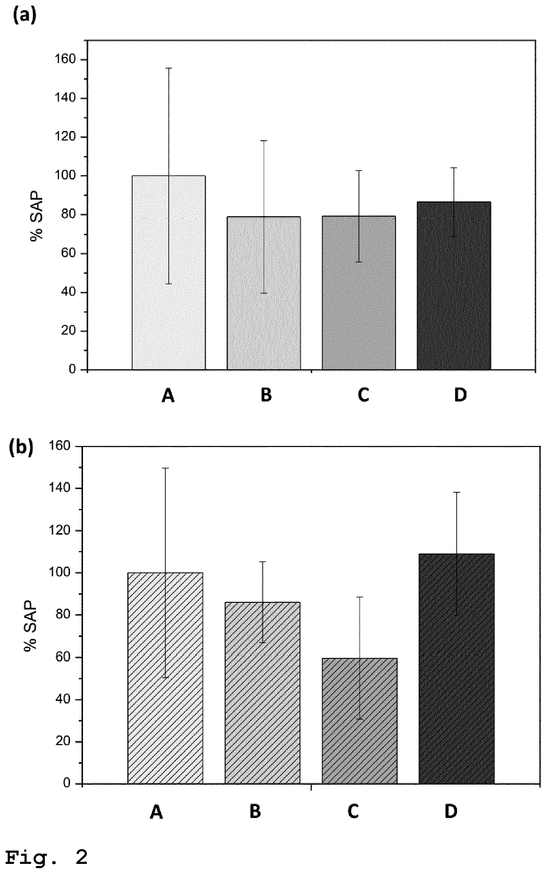

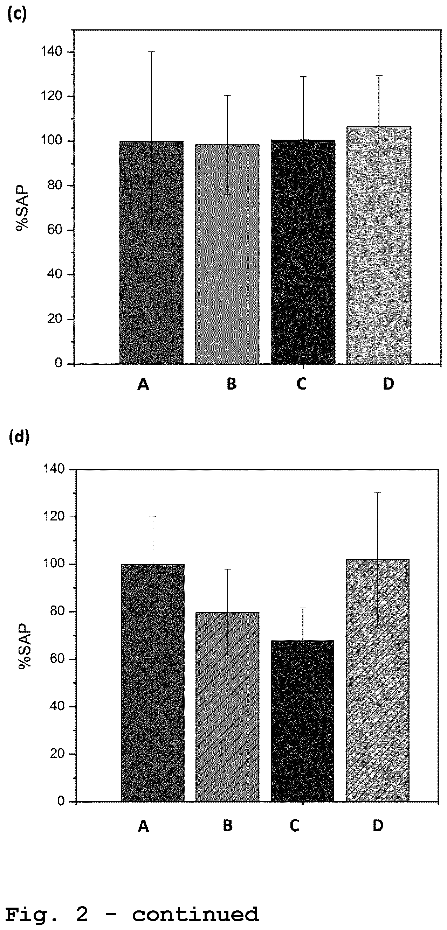

Fig. 2 shows the results from the SAP measurements in frozen pig skin samples that were treated with the lysate of the invention. (a) frozen skin, 2h pre-treatment before UV irradiation (A: untreated 2h, B: untreated 2h plus 5 min UV irradiation, C: placebo 2h plus 5 min UV irradiation, D: verum 2h plus 5 min UV irradiation); (b) frozen skin, 5h pre-treatment before UV irradiation (A: untreated 5h, B: untreated 5h plus 5 min UV irradiation, C: placebo 5h plus 5 min UV irradiation, D: verum 5h plus 5 min UV irradiation); (c) frozen skin, 2h post-treatment after UV irradiation (A: untreated 2h, B: untreated 2h plus 5 min UV irradiation, C: placebo 2h plus 5 min UV irradiation, D: verum 2h plus 5 min UV irradiation); (d) frozen skin, 5h post-treatment after UV irradiation (A: untreated 5h, B: untreated 5h plus 5 min UV irradiation, C: placebo 5h plus 5 min UV irradiation, D: verum 5h plus 5 min UV irradiation).

### EXAMPLES

The following examples describe certain preferred embodiments of the present invention. It is however to be noted that the invention is not limited to such embodiments.

### Example 1: Preparation of a L. kefiranofaciens ferment lysate

500 l of a medium for culturing *L. kefiranofaciens* was prepared as follows. Two phases were separately prepared (Phase I and II).

| Phase I | g/l |
|---|---|
| Water | 703.612 |
| Yeast Peptone | 18.0 |
| Yeast extract | 3.6 |
| Potassium hydrogen phosphate, di- | 1.8 |
| Sodium acetate | 4.5 |
| Ammonium citrate tribasic | 1.8 |
| L-Cysteine hydrochloride monohydrate | 0.225 |

| Phase II | g/l |
|---|---|
| Water | 120.0 |
| MgSO₄·7H₂O | 0.018 |
| MnCl₂·4H₂O | 0.045 |

| | |
|---|---|
| Glucose monohydrate | 39.6 |
| Vitamin B12 Solution | 1.8 |

Phase I of the culture medium was sterilized by autoclavation. Phase II is heat-sensitive and is therefore subjected to sterile filtration using a standard filter membrane for medium sterilization. After sterilization, phase I and phase II were mixed in a 600 l fermenter, and a pH of 5.5 was adjusted. The medium was inocculated with 0.5% of a *L. kefiranofaciens* strain and cultured at 32°C anaerobically in a N₂ atmosphere (less than 0.3% oxygen) under gentle stirring for about 8 days until the glucose in the medium is completely consumed.

At the end of culturing, the cell number is determined. If necessary, the cell number was set to 1×10⁸ cells/ml to 1×10⁹ cells /ml either by diluting or concentrating the content of the fermenter. Then, the fermenter is heated to 85°C for 30 minutes. Subsequently, the culture is harvested, and the cells are lysed by subjecting the complete content of the fermenter to high pressure homogenization using 2 cycles at 850 bar in a Ariete 2 High Pressure Homogenizer (GEA, Düsseldorf, Germany). The resulting ferment lysate is used for the skin studies described below.

### Example 2: Dermatological Study 1

In a first dermatological study (Study 1), the effectiveness of the ferment lysate prepared as described in Example 1 was examined. 3% (w/w) of the lysate was included in a standard cosmetic cream composition (verum) and compared with the standard cream composition as a control.

Both products were applied to the face twice daily by 10 test subjects over a period of four weeks. The test participants were instructed not to use an equivalent product in the test area during the test period during the test period.

After the four-week period, the skin of the test subjects was analyzed in terms of skin elasticity, skin firmness and transepidermal water loss (TEWL).

### Measurement of skin elasticity and skin firmness

The skin is viscoelastic organ due to structural proteins such as collagen and elastin. Skin elasticity and firmness was measured with a Cutometer^{®} dual MPA 580 (Courage & Khazaka electronic GmbH). This device uses the so-called suction method. A certain negative pressure is generated in the measuring head for a defined period of time and the penetration depth of the skin is measured without contact using an optical method. The light intensity is influenced by the penetration depth of the skin and automatically converted into mm (accuracy ± 3%) . This measuring principle makes it possible to measure various parameters. The most meaningful are the parameters R0 (skin firmness, ability of the skin of not being sucked in), R2 (elasticity of the skin, ability to return to its original state) and R8 (total regression after the pressure is turned off). The smaller R0 is, the firmer the skin is. The closer R8 is to R0 (R2Max = 1), i.e. the larger R2 is, the more elastic the skin is. At least three measurements were carried out at different points in the test area at each measurement time. An untreated area of skin is used as a control area.

### Measurement of transepidermal water loss (TEWL)

On average, every person loses half a liter of water through the skin every day. This diffusion is part of a healthy metabolism. Passive water loss through diffusion is referred to as transepidermal water loss (TEWL) and reflects the functionality of the skin barrier. Thus, a disturbed skin barrier shows an increased TEWL. The TEWL is measured with the Tewameter^{®} TM 300 (Courage + Khazaka electronic GmbH). The Tewameter determines the water vapor gradient over a defined area of skin. The physical basis for the measurement is Fick's first law of diffusion. It states how many particles of a substance move through a unit area perpendicular to the direction of diffusion per unit time. The measuring head of the Tewameter consists of a hollow cylinder so as not to influence the microclimate during the measurement. Two pairs of probes, a humidity and a temperature sensor, are mounted at different heights in the hollow cylinder and determine the mass of water (g) per time (h) and area (m²). The measurement result is output accordingly in g/h/m². Before the measurement, the probe is warmed up with the probe warmer PR 100 (Courage + Khazaka electronic GmbH) to 32°C to ensure stable measurements. At least three measurements are taken at different points in the test area at each measurement time. An untreated area of skin is used as a control area.

Results: From a clinical-dermatological point of view, there were no relevant skin reactions in the test area and the product was very well tolerated. Neither irritation nor allergic reactions were observed.

It was found that the verum improved skin elasticity. An improvement of skin elasticity of 12.90 % was measured in the test area. The change in skin elasticity in the control area was 0.52 %. Skin firmness was also improved. Specifically, an improvement of skin firmness of 7.01 % was measured in the test area, while the change in the control area was 0.52 %.

### Example 3: Dermatological Study 2

In a second dermatological study (Study 2), the efficacy of the ferment lysate prepared as described in Example 1 was examined at two concentration (1% and 3%) on ex vivo human skin explants upon UV-A irradiation was assessed through the evaluation of carbonylation levels associated with oxidative damage. The lysate is referred to as F262 #22716-10 in this study.

Eighteen explants were obtained with the informed consent from abdominal surgery of a 41-year-old female Caucasian donor. After surgery, they were kept alive by culturing on metal grids into standard 12-well plates in contact in OxiProteomics^{®} medium at 37°C in 5% CO₂ humidified air.

After reception, each skin explant was distributed in 6 experimental groups (n=3 per condition, the culturing medium was renewed every 24 hours (Table 2).

**Table 2: Experimental Groups**

| **Batch** | **Description** | **Treatment** |
|---|---|---|
| 1 | Control | Not treated, not stressed |
| 2 | Stress (UV-A irradiation) | Exposed to UV-A (peak at 365nm, 6 J/cm²) |
| 3 | #22716-10 at 1% + Stress | 1 appl. for 24 h, followed by exposure to UV-A |
| 4 | #22716-10 at 1% + Stress | 1 appl. for 24 h, followed by exposure to UV-A |

For efficacy evaluation, products were topically applied for 24h on the skin explant's surface (2 mg/cm²). 24 hours later, medium was replaced by 2 mL of Hank's Balanced Salt Solution (HBSS), and skin explants were irradiated with UV-A (LED source, emission peak at λ=365 nm; 6 J/cm²; 40 minutes of irradiation) using the OxiProteomics^{®} irradiation system. Just after the irradiation, skin explants were sampled, transferred in OCT for cryopreservation, snap-frozen in liquid nitrogen and conserved at -80°C until analyses.

### Detection, visualization, and quantification of Carbonyls

Explant sections of 5 µm of thickness were obtained using a cryostat (Leica) and fixed with a solution containing 95% Ethanol and 5% acetic acid. Oxidatively damaged (carbonylated) proteins were labeled using an OxiProteomics^{®} fluorescent probe (Ex = 647 nm / Em = 650 nm) functionalized to specifically bind to carbonyl moieties and DAPI (4',6-diamidino-2-phenylindol) for nuclear labeling. Fluorescent images were collected with an epi-fluorescent microscope (EVOS M5000 Imaging System) and analyzed with ImageJ software (Schneider, 2012). Image comparisons of different conditions were achieved using identical conditions of acquisition, exposure time and objective (40X). The raw images were collected in a range of intensity of specific signal and analyzed with ImageJ software. The intensity of carbonylation was obtained by the integration of the specific fluorescence signal normalized by the evaluated area. In each image, the quantification of carbonylation level was independently obtained for the different anatomical compartments (dermis, epidermis and stratum corneum) and for the overall (total skin). Three images per conditions (1 image per explant) were used to quantify the carbonylation levels; the mean value and standard deviation were obtained.

Results: Under the test conditions applied in this study, the Stress exposure (UV-A) induced a significant increase of carbonylation level in the whole skin section and in both skin compartments, i.e. in the stratum corneum, epidermis and dermis). The test product efficiently preserved and protected the skin from the UV-A-induced increase of carbonyls.

In whole skin, the test product was found to have a protective efficacy of 50% (at 1%) and 81% (at 3%). In the stratum corneum the protective efficacy was 53% (at 1%) and 70% (at 3%). In the epidermis, the protective efficacy was 86% (at 1%) and 77% (at 3%). Finally, in the dermis, the protective efficacy was 87% (at 1%) and 42% (at 3%).

These results indicate beneficial effects of the test product on preserving tissular oxidation (carbonylation). Overall, the test product showed significant beneficial effects, preserving skin integrity from deleterious effects of UV-A exposure, and suggesting their potential in anti-aging skincare products The results of the study are shown in Figure 1.

### Example 4: Dermatological Study 3

In a third dermatological study (Study 3), the antioxidative potential (SAP) was measured in skin samples by electron spin resonance (ESR) spectroscopy. The sample was treated with a test composition that contained the ferment lysate prepared as described in Example 1. 3% (w/w) of the lysate was included in a standard cosmetic cream composition (verum) and compared with the standard cream composition as a control. The lysate is referred to as F262-2.01 in this study. The control is referred to as F255-5.01.

The skin has an intrinsic antioxidative protection due to many enzymatic and non-enzymatic antioxidant systems present in the epidermis. For measuring the SAP, the skin is labelled with an ESR active test radical that is reduced by the antioxidant systems inside the epidermis and dermis.

An active substance, applied to the stratum corneum and able to penetrate, will increase the SAP. A time dependent penetration profile of the tested substance can be obtained on skin biopsies labelled with a semi-stable free radical in a biopsies flat cell. The signal intensities are registered by ESR spectroscopy. Different formulations of an active substance can be tested regarding to their ability to enhance the SAP of the skin [1-3].

The SAP value, expressed in percentage, is a quantitative determination of the antioxidant activity inside the epidermal and dermal layers of the skin. If the SAP is above 100%, the topical applied actives were able to penetrate and are effective against free radical injury in the skin. On the other hand, if the SAP value is below 100%, the topical treatment (chemical or physical stress such as sun exposure or detergents) has diminished the skin's antioxidant defense system.

### Determination of SAP

Skin biopsies obtained from local slaughterhouse (pig skin, 6 months old animals, external lobe of the ears) were washed, the subdermal fat was removed, and the skin was cut in 1x1 cm pieces. The samples were frozen until further use. After thawing, the test composition was applied on the epidermal layer of the skin sample. The skin pieces were placed on a filter paper saturated with the test radical (TEMPO, 2,2,6,6-tetramethyl piperidine-N-oxyl, Sigma-Aldrich, Munich, Germany) at 1 mM concentration in water for 5 minutes. A skin biopsy of 4 mm was taken, placed in a special ESR tissue cell and ESR spectra of the test radical were recorded after different times. The recording ESR parameters were the following: 50 G sweep width, 200 Gain, 1 G modulation amplitude, 20 mW attenuation, 3358 G central field, 0.14 sec time constant. The Irradiation source was an UV solar simulator 300 W Oriel (New-port). The irradiances as integrated value over the spectral ranges were E (UVB=280-320) = 23,5 W/m2 and E (UVA = 320-400nm) = 180 W/m². The amplitudes of the ESR spectra were plotted against time and the obtained kinetics were fitted using a monoexponential decay. The kinetic parameter k of the untreated skin was set as 100%. The penetration time varied between 2h and 5h.

Results: The application of the verum induced an increase in the SAP in all verum samples tested. After 5 hours of application the skin showed higher SAP values after UV irradiation, indicating that the penetration of the actives into the skin is necessary to evolve the product's efficacy. The results of the SAP measurement are shown in Figure 2. It can be seen that in frozen skin samples that were treated with the lysate of the invention either 2h (a) or 5h (b) before UV irradiation, the %SAP increased and reached the level of the untreated sample again. Essentially the same results were observed in frozen skin samples that were treated with the lysate of the invention either 2h (a) or 5h (b) after UV irradiation.

Within these 2h and 5h time kinetics, it is clearly recognizable that a longer penetration time increases the effectiveness of the lysate. This means that the antioxidant effect occurs through penetration and triggering of intracellular mechanisms rather than a mere radical scavenging effect.

### REFERENCE LIST

[1] T. Herrling et al., Detection and influencing of the Antioxidative Potential (AOP) of human skin. SÖFW Journal 7 (1996) 472-476.
[2] K. Jung et al. Antiaging status of skin characterized by the Skin Antioxidative Protection (SAP) - efficacy of topically applied antioxidants. SÖFW journal 9 (2006).
[3] K. Jung & Th. Herrling. Insights in the role of antioxidants in modern skin and hair care products - methods to prove their activity. Global Ingredients and Formulations Guide 2007 Verlag f. chem. Industrie, Augsburg, Germany, p. 49-67.
[4] Y. Wang et al., Complete genome sequence of Lactobacillus kefiranofaciens ZW3. J. Bacteriol. 2011, 193: 4280-4281.
[5] T. Fujisawa et al., Lactobacillus kefiranofaciens sp. nov. isolated from kefir grains. Int. J. Syst. Bacteriol. 1988, 38: 12-14.
[6] J. Zheng et al., A taxonomic note on the genus Lactobacillus: Description of 23 novel genera, emended description of the genus Lactobacillus Beijerinck 1901, and union of Lactobacillaceae and Leuconostocaceae. Int. J. Syst. Evol. Microbiol. 2020, 70: 2782-2858.
[7] M. Vancanneyt, et al., Reclassification of Lactobacillus kefirgranum Takizawa et al. 1994 as Lactobacillus kefiranofaciens subsp. kefirgranum subsp. nov. and emended description of L. kefiranofaciens Fujisawa et al. 1988. Int. J. Syst. Evol. Microbiol. 2004, 54: 551-556.
[8] R. Enikeev, Development of a new method for determination of exopolysaccharide quantity in fermented milk products and its application in technology of kefir production. Food Chemistry. 2012, 134(4), 2437-2441.
[9] M. Georgalaki, Lactobacillus kefiranofaciens: From Isolation and Taxonomy to Probiotic Properties and Applications. Microorganisms. 2021, 9(10): 2158.
[10] A.A. Bengoa et al., Kefir micro-organisms: Their role in grain assembly and health properties of fermented milk. J. Appl. Microbiol. 2018, 126: 686-700.

## Claims

1. Cosmetic skin care composition comprising non-viable components of bacterial cells, wherein said cells belong to the species *Lactobacillus kefiranofaciens.*

2. Cosmetic composition of claim 1, wherein the composition comprises a cell lysate of *Lactobacillus kefiranofaciens.*

3. Cosmetic composition of claim 2, wherein the lysate is a ferment lysate.

4. Cosmetic composition of any of claims 1-3, wherein the lysate is present in the composition in an amount of 0.1 to 15.0% (w/w), and preferably 0.5 to 5.0% (w/w).

5. Cosmetic composition according to any of claims 1-4, wherein said composition is formulated for topical application.

6. Cosmetic composition of claim 5, wherein the said composition is formulated for topical application to the skin of the face.

7. Cosmetic composition according to any of claims 1-6, wherein said composition is formulated as an ointment, cream, lotion, paste, gel, hydrogel, foam or powder.

8. Cosmetic composition according to any of claims 1-7, wherein the components of the bacterial cells are derived from a *Lactobacillus kefiranofaciens* strain that produces the exopolysaccharide kefiran.

9. A cosmetic method of improving skin appearance, comprising administering a composition of any of claims 1-8 to the skin of said subject.

10. A cosmetic method of improving skin elasticity, comprising administering a composition of any of claims 1-8 to the skin of said subject.

11. A cosmetic method of reducing transepidermal water loss, comprising administering a composition of any of claims 1-8 to the skin of said subject.

12. A cosmetic method of preventing the signs of aging, comprising administering a composition of any of claims 1-8 to the skin of said subject.

13. A cosmetic method of any of claims 9-12, wherein said administration comprises topical administration to the skin of the face or body of said subject.

14. Use of a composition of any of claims 1-8 for cosmetic skin care.

15. Use of a composition of any of claims 1-8 for improving skin appearance, improving skin elasticity, reducing transepidermal water loss, or preventing the signs of aging.
